# FASCICULE DE BREVET EUROPEEN

(11) **EP 0 910 787 B1**
(45) Date de publication et mention de la délivrance du brevet: **19.11.2008**
(21) Numéro de dépôt: 98920616.4
(22) Date de dépôt: 14.04.1998
(51) Int. Cl.: G01N 1/14, C12M 1/28

(54) **PROCEDE ET DISPOSITIF DE REMPLISSAGE AVEC UN MILIEU LIQUIDE D'UNE CARTE D'ANALYSE**
VERFAHREN UND VORRICHTUNG ZUM FÜLLEN EINER TESTKARD MIT FLÜSSIGKEITEN
METHOD AND DEVICE FOR FILLING AN ANALYSIS CARD WITH A LIQUID MEDIUM

(30) Priorité: 15.04.1997 FR 9704851
(43) Date de publication de la demande: 28.04.1999
(73) Titulaire: BIO MERIEUX, 69280 Marcy l'Etoile (FR)
(72) Inventeur: COLIN, Bruno, F-69280 Marcy L'Etoile (FR); JARAVEL, Cécile, F-69003 Lyon (FR)
(74) Mandataire: Guerre, Dominique
(86) Numéro de dépôt international: PCT/FR1998/000747
(87) Numéro de publication internationale: WO 1998/046977

(56) Documents cités:
- EP-A- 0 107 631
- EP-A- 0 745 856
- WO-A-88/07351
- WO-A-90/08308
- DE-A- 2 941 177
- DE-C- 4 429 739
- US-A- 3 963 355
- US-A- 4 018 652

## Description

L'invention concerne le remplissage avec un milieu liquide d'une carte d'analyse.

Par "carte d'analyse", on entend tout moyen à usage unique, ou non, permettant d'effectuer une ou plusieurs analyses simultanées, à partir d'un seul et même échantillon et d'un ou plusieurs réactifs, et ce de manière étanche par rapport au milieu extérieur, le résultat de la ou des analyses étant obtenu par exemple par voie optique. De manière générale, une telle carte d'analyse comprend un corps dans lequel est formée au moins une cavité de lecture, recevant lors de l'utilisation la totalité ou une quote-part de l'échantillon avec au moins un réactif préexistant dans ladite cavité, ainsi qu'un orifice communiquant avec ladite cavité par au moins un canal interne. Cette ou ces cavités communiquent donc avec l'extérieur uniquement par l'orifice précité, tandis que la carte est en général associée ou coopère avec un conduit externe, souple, communiquant de manière étanche avec cet orifice. C'est ce conduit externe qui permet de prélever comme décrit ci-après l'échantillon du milieu liquide à analyser.

De telles cartes sont bien connues par l'art antérieur, et notamment par les brevets américains 3 963 355, 4 018 652 et 4 038 151, ou encore le brevet européen EP 0 745 856, auxquels on se réfèrera en tant que de besoin.

Par "milieu liquide", on entend tout produit ou corps liquide, en tout cas susceptible de s'écouler par pompage, quelle que soit la viscosité ou fluidité de ce liquide; en particulier, s'agissant d'une analyse biologique, on entend par milieu liquide un milieu comportant par exemple un ou des microorganismes ou un produit biologique à analyser.

Le document WO-A-88/07351 décrit un dispositif, ainsi qu'un procédé de mise sous vide de tubes fermés, destinés à la collecte de fluide corporel.

Le document DE-A-2941177 décrit un dispositif pour l'absorption de prélèvements liquides ou gazeux contenus dans un réservoir, à l'aide d'un récipient sous vide, dont l'orifice d'admission est obstrué avec un bouchon élastique, et d'une sonde qui transperce ledit bouchon et qui est reliée au réservoir.

S'agissant du remplissage d'une carte d'analyse telle que précédemment définie, jusqu'à présent, on a procédé de la manière suivante
- on dispose d'un caisson étanche, avec des moyens de contrôle de la pression interne, et notamment du vide lorsque ledit caisson a été mis sous dépression;
- on dispose dans le caisson, d'une part un contenant, par exemple une éprouvette, dans lequel est disposé le milieu liquide à échantillonner ou prélever, et d'autre part à côté du contenant la carte d'analyse avec l'extrémité libre du conduit externe, immergée dans le milieu liquide contenu par le contenant;
- on évacue l'air du caisson, jusqu'à un niveau de vide significatif, par exemple inférieur à 100 mbar absolus, moyennant quoi on évacue l'air de la carte d'analyse, et l'air évacué traverse le milieu liquide du contenant;
- après rupture du vide, et en ramenant le caisson à la pression atmosphérique, le milieu liquide se trouve aspiré dans la carte d'analyse jusqu'à la ou les cavités de lecture, ainsi remplies.

Un tel procédé apparaît particulièrement contaminant, puisque le barbotage intervenant lors de la mise sous vide est susceptible de projeter à l'extérieur du contenant, et donc ultérieurement au contact de l'utilisateur ou manipulateur, des particules ou gouttelettes du milieu liquide, et ce malgré toutes les précautions pouvant être prises à cet égard. S'agissant d'un milieu liquide contenant un agent pathogène, ceci n'apparaît pas satisfaisant.

La présente invention a pour objet un mode de remplissage d'une carte d'analyse, non contaminant, en ce qu'il travaille toujours sous dépression, c'est-à-dire sans possibilité d'évacuer vers l'extérieur le milieu liquide manipulé.

Conformément à la présente invention, successivement :
- pour évacuer le gaz contenu dans la carte d'analyse, on connecte directement l'extrémité libre du conduit externe ouvert, de manière étanche, avec un moyen d'évacuation, et ce en dehors du milieu liquide manipulé;
- on obture le conduit externe, pour maintenir la dépression dans la carte d'analyse;
- on immerge l'extrémité libre du conduit externe obturé dans le milieu liquide;
- et on réouvre le conduit externe après immersion de cette extrémité libre, pour aspirer le milieu liquide dans la carte d'analyse.

La présente invention apporte de plus l'avantage déterminant selon lequel le pompage du gaz contenu dans la carte d'analyse exige un travail limité, et devient par conséquent compatible avec des moyens de pompage relativement simples, voire rudimentaires, en particulier manuels. De plus, le mode de remplissage précédemment décrit peut être utilisé aussi bien de manière manuelle, qu'au sein d'un automate d'analyse.

La présente invention est maintenant décrite par référence au dessin annexé, dans lequel:
- la Figure 1 représente en perspective et de manière schématique, une carte d'analyse telle que considérée par la présente invention;
- la Figure 2 représente de manière schématique un dispositif de remplissage conforme à l'invention;
- la Figure 3 représente en perspective un mode de réalisation du dispositif schématisé à la Figure 2;
- et la Figure 4 représente une vue en coupe du moyen de connexion et du moyen d'obturation faisant partie du dispositif représenté à la Figure 3.

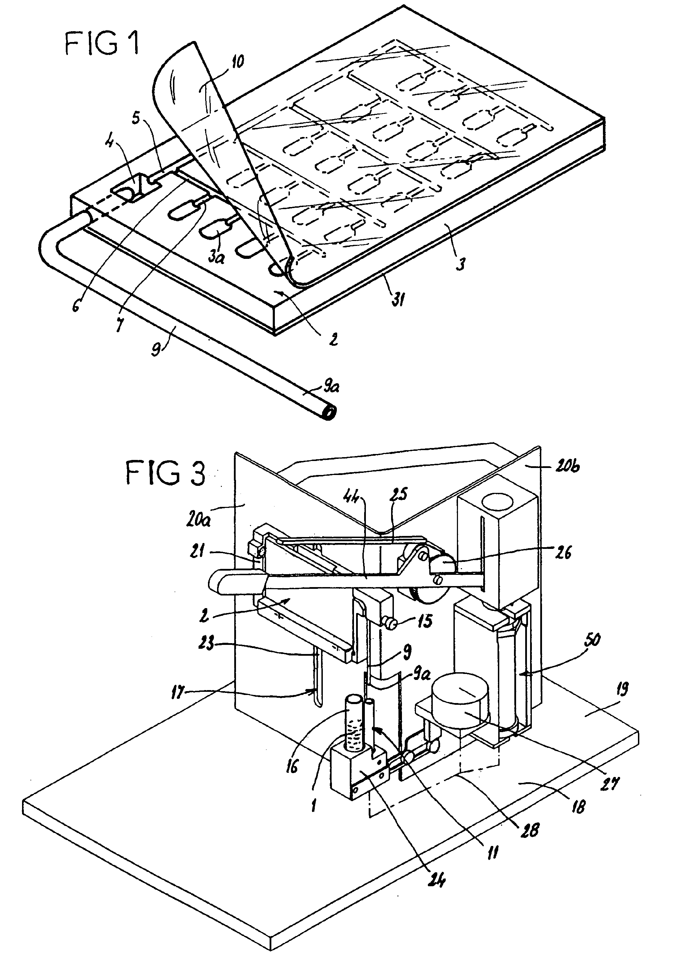

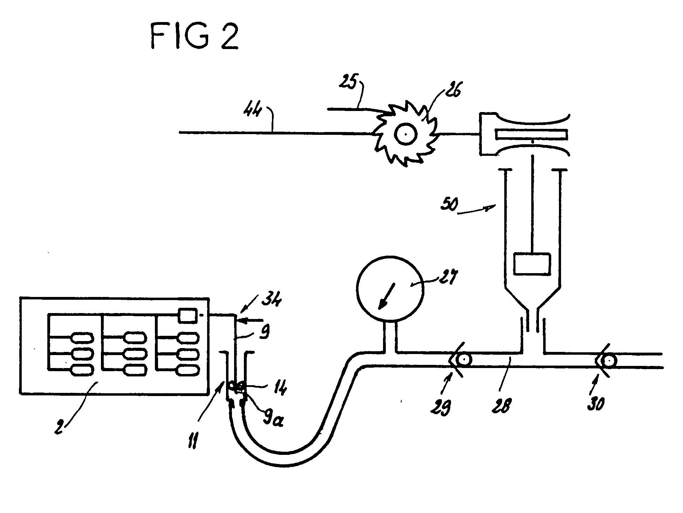

Une carte d'analyse 2, traitée par le procédé de remplissage selon l'invention, avec le dispositif représenté aux Figures 2 et 3, comprend :
- un corps 3, en matière plastique, moulé, de forme générale plate et rectangulaire, dans lequel sont formés d'une part plusieurs séries de cavités 3a de lecture, et d'autre part des canaux ouverts internes 7, ainsi qu'un orifice 4;
- deux feuilles 10 de matière plastique transparente, collées de manière étanche respectivement sur les deux faces du corps 3;
- et un conduit externe 9, souple, ayant la forme d'une paille coudée, assemblé de manière étanche avec le corps 3, au moment de l'utilisation de la carte d'analyse, comportant une extrémité libre 9a, et une extrémité opposée communiquant de manière étanche, après montage ou assemblage, avec l'orifice 4.

S'agissant de la circulation du liquide dans la carte 2, un conduit distributeur 5 communique d'un côté avec l'orifice 4, et de l'autre côté avec une série de ramifications 6, sur chacune desquelles les différentes cavités 3a de lecture sont branchées par des canaux internes 7 respectifs.

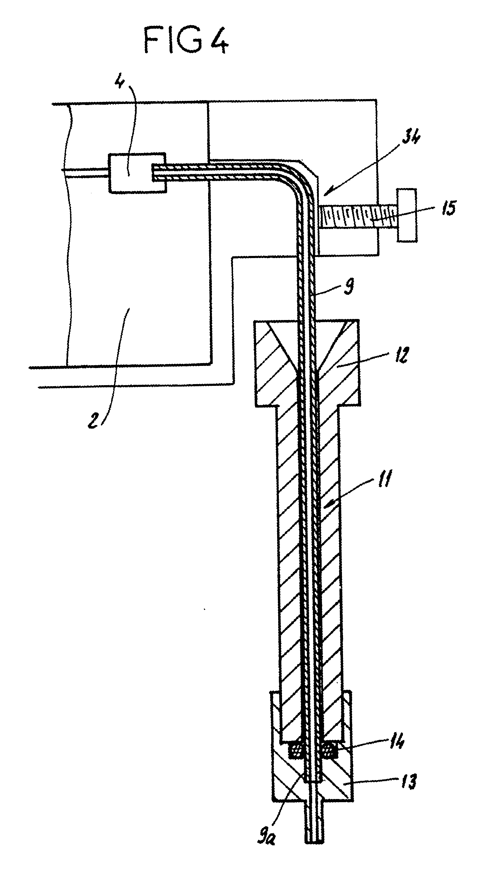

Conformément à la Figure 3, un dispositif de remplissage conforme à l'invention comprend :
- un bâti 18 comportant un socle 19 et une paroi verticale comprenant deux flancs 20a et 20b à angle droit;
- un chariot 21, adapté pour recevoir et maintenir la carte d'analyse 2 en position verticale, avec le conduit externe 9 en position également verticale; ce chariot est susceptible de coulisser contre le flanc 20a, de manière contrôlée, grâce à un moyen de déplacement 17, et notamment une glissière 23;
- un autre chariot 24, monté de manière coulissante, au bas du flanc 20b; ce chariot est adapté pour supporter le tube ou contenant 16 dans lequel est disposé le milieu liquide 1 à prélever, ainsi que le moyen de connexion 11 décrit ci-après;
- un moyen d'évacuation 50 consistant en une pompe manuelle alternative, montée sur le flanc 20b, actionnée par un levier 44, pouvant être bloqué en rotation par un mécanisme à cliquet 26 commandé par un bras 25;
- un moyen de connexion 11, plus particulièrement représenté à la Figure 4, comprenant d'un côté un embout 12 d'accouplement étanche et amovible avec l'extrémité libre 9a du conduit externe 9, grâce à un joint torique 14, serré entre ledit embout et un contre-embout 13, ce dernier communiquant directement de l'autre côté avec le moyen d'évacuation 50;
- un moyen 34 d'obturation et réouverture du conduit externe 9, solidaire du chariot 21; ce moyen consiste en une vis 15, permettant de pincer le conduit 9; il peut être remplacé par un robinet ;
- un manomètre 27 de mesure-de la dépression;
- un circuit 28, muni de clapets 29 et 30 (Fig. 2) à la sortie du moyen d'évacuation 50, permettant une communication entre la sortie de la pompe et l'entrée du moyen de connexion 11.

Avec le dispositif précédemment rempli, le procédé de remplissage suivant peut être pratiqué :
- on dispose dans le chariot 21 la carte d'analyse 2 à remplir, et on laisse le moyen d'obturation 34 ouvert;
- on déplace les chariots 21 et 24 par le biais du moyen de déplacement 17, en sorte que l'extrémité libre 9a du conduit externe 9 communique de manière étanche avec le moyen de connexion 11 (conférer représentation de la Figure 4);
- en actionnant le levier 44, on évacue le gaz contenu dans la carte d'analyse 2, en l'occurrence l'air atmosphérique, et on contrôle la pression d'évacuation au moyen du manomètre 27; ceci peut requérir un ou plusieurs allers et retours en rotation du levier 44, selon la cylindrée du moyen d'évacuation 50; une fois le niveau souhaité de vide atteint, le levier est fixé en position au moyen du mécanisme à cliquet 26;
- dès cet instant, au moyen de la vis 15, on obture de manière étanche le conduit externe 9;
- par un nouveau mouvement des chariots 21 et 24, on amène l'extrémité libre 9a du conduit externe 9 dans le contenant 16, avec immersion de cette extrémité 9a dans le milieu liquide 1 contenu dans le contenant 16;
- on réouvre le conduit externe 9, après immersion de cette extrémité libre, en maintenant immergée l'extrémité 9a, ce qui provoque l'aspiration du milieu liquide 1 dans la carte d'analyse, jusqu'à remplir chacune des cavités de lecture 3a;
- et on scelle l'extrémité du conduit externe 9, opposée à l'extrémité libre 9a, en séparant et jetant le reste du conduit.

Dès cet instant, la carte d'analyse se trouve remplie et est prête pour subir le processus d'analyse requis, avec lecture des résultats au travers des différentes cavités 3a de lecture.

## Revendications

1. Procédé de remplissage avec un milieu liquide (1), d'une carte d'analyse (2) comprenant un corps (3) dans lequel est formée au moins une cavité (3a) de lecture, et un orifice (4) communiquant avec ladite cavité par au moins un canal interne (7), avec un conduit externe (9) communiquant avec ledit orifice, procédé selon lequel on évacue le gaz contenu dans la carte (2) par ledit conduit externe (9), puis on introduit le liquide (1) dans la carte (2) évacuée, avec immersion de l'extrémité libre (9a) dudit conduit externe (9) dans le milieu liquide (1), **caractérisé en ce que**, successivement
- pour évacuer le gaz contenu dans la carte d'analyse (2), on connecte directement l'extrémité libre (9a) du conduit externe (9) ouvert, de manière étanche, avec un moyen d'évacuation (50), en dehors du milieu liquide (1);
- on obture (34) le conduit externe (9), pour maintenir la dépression dans la carte d'analyse (2) ;
- on immerge l'extrémité libre (9a) du conduit externe obturé dans le milieu liquide (1)
- et on réouvre le conduit externe (9), après immersion de ladite extrémité libre (9a), pour aspirer le milieu liquide (1) dans la carte d'analyse (2).

2. Procédé selon la revendication 1, selon lequel le milieu liquide (1) est contenu dans un contenant (16), **caractérisé en ce que** successivement on connecte l'extrémité libre (9a) du conduit externe (9) ouvert avec le moyen d'évacuation (50), en dehors du contenant, on plonge ladite extrémité libre (9a) dudit conduit externe (9) obturé dans le milieu liquide (1) dans ledit contenant, et on réouvre le conduit externe (9) en maintenant ladite extrémité libre (9a) immergée dans ledit contenant.

3. Procédé selon la revendication 1, selon lequel le conduit externe (9) est un conduit souple, **caractérisé en ce que** l'obturation est obtenue par pincement dudit conduit, et la réouverture de ce dernier, en libérant ledit pincement.

4. Procédé selon la revendication 1, **caractérisé en ce que** le moyen d'évacuation (50) consiste en un moyen de pompage, par exemple de pompage manuel.

5. Dispositif de remplissage avec un milieu liquide (1), d'une carte d'analyse (2) comprenant un corps (3) dans lequel est formée au moins une cavité (3a) de lecture, et un orifice (4) communiquant avec ladite cavité par au moins un canal interne (7), avec un conduit externe (9) communiquant avec ledit orifice, ce dispositif comprenant un moyen d'évacuation (50), **caractérisé en ce qu'**il comprend
- un moyen de connexion (11), comportant d'un côté un embout (12) d'accouplement étanche et amovible avec l'extrémité libre (9a) du conduit externe (9) ouvert, et communiquant directement de l'autre côté avec le moyen d'évacuation (50);
- un moyen (34) d'obturation et réouverture du conduit externe.

6. Dispositif selon la revendication 5, **caractérisé en ce que** le moyen d'évacuation (50) est un moyen de pompage alternatif, notamment manuel.

7. Dispositif selon la revendication 5, **caractérisé en ce que** le moyen d'obturation (34) et de réouverture est un moyen de pincement, ou un robinet.

8. Dispositif selon la revendication 5, selon lequel le milieu liquide (1) est contenu dans un contenant (16), **caractérisé en ce qu'**il comporte un moyen (17) de déplacement de la carte d'analyse, d'une première position dans laquelle l'extrémité libre (9a) du conduit externe est accouplée de manière étanche avec le moyen de connexion (11), à une deuxième position dans laquelle ladite extrémité libre (9a) est immergée dans le milieu liquide (1) contenu dans le contenant (16).

## Claims

1. Method for filling, with a liquid medium (1), an analysis card (2) comprising a body (3) in which at least one reading cavity (3a) is formed, and an orifice (4) communicating with said cavity via at least one internal channel (7), with an external conduit (9) communicating with said orifice, in accordance with which method the gas contained in the card (2) is evacuated via said external conduit (9), then the liquid is introduced into the evacuated card, with immersion of the free end (9a) of said external conduit (9) in the liquid medium, **characterized in that**, in succession:
- to evacuate the gas contained in the analysis card (2), the free end (9a) of the open external conduit (9) is connected in a leaktight manner directly to an evacuation means (50), outside the liquid medium;
- the external conduit (9) is closed (34) in order to maintain the negative pressure in the analysis card (2);
- the free end (9a) of the closed external conduit is immersed in the liquid medium;
- and the external conduit (9) is re-opened, after immersion of said free end (9a), in order to aspirate the liquid medium into the analysis card (2).

2. Method according to Claim 1, in accordance with which the liquid medium is contained in a receptacle (16), **characterized in that**, in succession, the free end (9a) of the open external conduit (9) is connected to the evacuation means (50), outside the receptacle, said free end of said closed external conduit is dipped into the liquid medium in said receptacle, and the external conduit is re-opened, maintaining said free end immersed in said receptacle.

3. Method according to Claim 1, in accordance with which the external conduit (9) is a flexible conduit, **characterized in that** the closure is obtained by pinching said conduit, and the re-opening of the latter is obtained by releasing said pinching.

4. Method according to Claim 1, **characterized in that** the evacuation means (50) consists of a pumping 5 means, for example a manual pumping means.

5. Device for filling, with a liquid medium (1), an analysis card (2) comprising a body (3) in which at least one reading cavity (3a) is formed, and an orifice (4) communicating with said cavity via at least one internal channel (7), with an external conduit (9) communicating with said orifice, this device comprising an evacuation means (50), **characterized in that** it comprises:
- a connecting means (11) including on one side a joining piece (12) for leaktight and removable coupling to the free end (9a) of the open external conduit, and communicating directly on the other side with the evacuation means (50);
- a means (34) for closing and re-opening the external conduit.

6. Device according to Claim 5, **characterized in that** the evacuation means (50) is a reciprocating pumping means, in particular manual.

7. Device according to Claim 5, **characterized in that** the means for closing (34) and re-opening is a pinching means, or a valve.

8. Device according to Claim 5, in accordance with which the liquid medium (1) is contained in a receptacle (16), **characterized in that** it includes a means (17) for displacement of the analysis card from a first position, in which the free end (9a) of the external conduit is coupled in a leaktight manner to the connecting means (11), to a second position in which said free end (9a) is immersed in the liquid medium (1) contained in the receptacle (16).

## Patentansprüche

1. Verfahren zum Füllen einer Testkard (2) mit einem flüssigen Medium (1) mit einem Körper (3), in dem mindestens ein Lesehohlraum (3a) ausgebildet ist sowie eine Öffnung (4), die mit diesem Hohlraum über mindestens einen inneren Kanal (7) in Verbindung steht, und mit einer äußeren Leitung (9), die mit der Öffnung in Verbindung steht, wobei bei dem Verfahren das in der Kard (2) enthaltene Gas über die äußere Leitung (9) evakuiert wird, wonach die Flüssigkeit (1) durch Eintauchen des freien Endes (9a) der äußeren Leitung (9) in das flüssige Medium (1) in die evakuierte Kard (2) eingeführt wird, **dadurch gekennzeichnet, dass** nacheinander folgende Schritte durchgeführt werden:
- zur Evakuierung des in der Testkard (2) enthaltenen Gases wird das freie Ende (9a) der offenen äußeren Leitung (9) direkt dicht an ein Evakuierungsmittel (50) außerhalb des flüssigen Mediums (1) angeschlossen,
- die äußere Leitung (9) wird verschlossen (34), um den Unterdruck in der Testkard (2) aufrechtzuerhalten,
- das freie Ende (9a) der verschlossenen äußeren Leitung wird in das flüssige Medium (1) eingetaucht und
- nach dem Eintauchen des freien Endes (9a) wird die äußere Leitung (9) wieder geöffnet, um das flüssige Medium (1) in die Testkard (2) zu saugen.

2. Verfahren nach Anspruch 1, wobei das flüssige Medium (1) in einem Behälter (16) enthalten ist, **dadurch gekennzeichnet, dass** nacheinander das freie Ende (9a) der offenen äußeren Leitung (9) außerhalb des Behälters an das Evakuierungsmittel (50) angeschlossen wird, das freie Ende (9a) der verschlossenen äußeren Leitung (9) in das flüssige Medium (1) in dem Behälter eingetaucht wird und die äußere Leitung (9) wieder geöffnet wird, während das freie Ende (9a) im Behälter eingetaucht gehalten wird.

3. Verfahren nach Anspruch 1, wobei es sich bei der äußeren Leitung (9) um eine flexible Leitung handelt, **dadurch gekennzeichnet, dass** das Verschließen durch Kneifen der Leitung und ihr erneutes Öffnen durch Freigeben erzielt wird.

4. Verfahren nach Anspruch 1, **dadurch gekennzeichnet, dass** das Evakuierungsmittel (50) aus einem Pumpmittel, beispielsweise einem Mittel zum manuellen Pumpen, besteht.

5. Vorrichtung zum Füllen einer Testkard (2) mit einem flüssigen Medium (1) mit einem Körper (3), in dem mindestens ein Lesehohlraum (3a) ausgebildet ist sowie eine Öffnung (4), die mit diesem Hohlraum über mindestens einen inneren Kanal (7) in Verbindung steht, und mit einer äußeren Leitung (9), die mit der Öffnung in Verbindung steht, wobei diese Vorrichtung ein Evakuierungsmittel (50) umfasst, **dadurch gekennzeichnet, dass** sie
- ein Anschlussmittel (11), das auf der einen Seite einen Stutzen (12) zum dichten und entfernbaren Ankoppeln an das freie Ende (9a) der offenen äußeren Leitung (9) aufweist und auf der anderen Seite direkt mit dem Evakuierungsmittel (50) in Verbindung steht, und
- ein Mittel (34) zum Verschließen und erneuten Öffnen der äußeren Leitung umfasst.

6. Vorrichtung nach Anspruch 5, **dadurch gekennzeichnet, dass** es sich bei dem Evakuierungsmittel (50) um ein alternierendes Pumpenmittel, besonders ein manuelles, handelt.

7. Vorrichtung nach Anspruch 5, **dadurch gekennzeichnet, dass** es sich bei dem Mittel (34) zum Verschließen und erneuten Öffnen um ein Klemmmittel oder einen Hahn handelt.

8. Vorrichtung nach Anspruch 5, wobei das flüssige Medium (1) in einem Behälter (16) enthalten ist, **dadurch gekennzeichnet, dass** sie ein Mittel (17) zum Verschieben der Testkard von einer ersten Position, in der das freie Ende (9a) der äußeren Leitung dicht an das Anschlussmittel (11) angekoppelt ist, in eine zweite Position, in der das freie Ende (9a) in das im Behälter (16) enthaltene flüssige Medium (1) eingetaucht ist, aufweist.
